# EUROPEAN PATENT SPECIFICATION

(11) **EP 4 172 653 B1**
(45) Date of publication and mention of the grant of the patent: **06.08.2025**
(21) Application number: 21736554.3
(22) Date of filing: 22.06.2021
(51) Int. Cl.: G01S 7/52, A61B 8/00, G01S 15/89

(54) **ANALOG CONTINUOUS WAVE DOPPLER ULTRASOUND SIGNAL GENERATION WITHIN AN ULTRASOUND PROBE AND ASSOCIATED SYSTEMS, DEVICES, AND METHODS**
ERZEUGUNG EINES ANALOGEN DOPPLER-ULTRASCHALLSIGNALS MIT KONTINUIERLICHER WELLE IN EINER ULTRASCHALLSONDE UND ZUGEHÖRIGE SYSTEME, VORRICHTUNGEN UND VERFAHREN
GÉNÉRATION DE SIGNAL ULTRASONORE DOPPLER À ONDE CONTINUE ANALOGIQUE À L'INTÉRIEUR D'UNE SONDE ULTRASONORE ET SYSTÈMES, DISPOSITIFS ET PROCÉDÉS ASSOCIÉS

(30) Priority: 24.06.2020 US 202063043286 P
(43) Date of publication of application: 03.05.2023
(73) Proprietor: Koninklijke Philips N.V., 5656 AG Eindhoven (NL)
(72) Inventor: SAVORD, Bernard, Joseph, 5656 AE Eindhoven (NL)
(74) Representative: Philips Intellectual Property & Standards
(86) International application number: PCT/EP2021/066921
(87) International publication number: WO 2021/259900

(56) References cited:
- US-A1- 2016 097 846
- BARROSO ERNESTO CARRILLO ET AL: "Doppler ultrasonic system for flow measurement in patients with Diabetic Foot using reconfigurable logic and Wishbone architecture", 2017 GLOBAL MEDICAL ENGINEERING PHYSICS EXCHANGES/PAN AMERICAN HEALTH CARE EXCHANGES (GMEPE/PAHCE), IEEE, 20 March 2017 (2017-03-20), pages 1 - 6, XP033116360, DOI: 10.1109/GMEPE-PAHCE.2017.7972099
- LIM JAEMYUNG ET AL: "Highly-integrated guidewire vascular ultrasound imaging system-on-a-chip", 2018 IEEE CUSTOM INTEGRATED CIRCUITS CONFERENCE (CICC), IEEE, 8 April 2018 (2018-04-08), pages 1 - 4, XP033339092, DOI: 10.1109/CICC.2018.8357032

## Description

### TECHNICAL FIELD

The present disclosure relates generally to ultrasound imaging, such as continuous wave (CW) Doppler imaging. In particular, analog CW Doppler signals are generated with in-phase and quadrature (I/Q) mixers within an ultrasound transducer probe.

### BACKGROUND

Ultrasound imaging systems are often used for medical imaging. An ultrasound imaging system typically includes a transducer probe as well as a main processing system. The transducer probe may include an array of ultrasound transducer elements. The ultrasound transducer elements send acoustic waves through a patient's body and generate signals as the acoustic waves are reflected back by tissues and/or organs within the patient's body. In traditional ultrasound applications, the timing and/or strength of the echo signals may correspond to the size, shape, and mass of the tissues, organs, or other features of the patient and images depicting the measured tissues, organs, or other features may be displayed to a user of the ultrasound system. Some ultrasound applications additionally employ continuous wave (CW) Doppler imaging methods to measure velocities within a patient, such as velocities of fluids (e.g. blood flow). Analog ultrasound echo signals corresponding to each ultrasound transducer element may be passed through a cable from the transducer probe to the main processing system. For CW Doppler applications, the processing system may display a graphical representation of velocities within the patient.

To transmit analog ultrasound echo signals from the probe to the main processing system, the connecting cable may include many conductors and, in some instances, may require a conductor or set of conductors for each receiving ultrasound transducer element, making the cable very thick, unwieldy, complex, and cumbersome. Due to the many conductors within the cable, the cost of the cable can also be the costliest component in an ultrasound imaging system. The cable may also have a high failure rate.

One approach to overcoming the limitations of transmitting analog ultrasound signals from the probe to the processing system is to include low-power analog-to-digital converters (ADCs) in the transducer probe, perform full or partial beamforming and/or multiplexing, and then transmit digital signals via a reduced number of conductors. This method may significantly reduce the cost, diameter, and overall maneuverability of the cable connecting the ultrasound imaging probe and the main processing system. However, due to the high dynamic range of CW Doppler ultrasound signals, in some embodiments, this approach may be unsuitable for ultrasound systems with a CW Doppler path. In particular, the low-power ADCs used to convert analog signals to digital signals may not have sufficient dynamic range to convert analog signals associated with CW Doppler imaging and maintain high signal quality.

"Doppler ultrasonic system for flow measurement in patients with Diabetic Foot using reconfigurable logic and Wishbone architecture" by E. Carrillo Barroso et al, 2017 Global Medical Engineering Physics Exchanges/Pan American Health Gare Exchanges (GMEPE/PAHCE), IEEE, 20 March 2017, pp. 1-6 (XP033116360), presents some results in the design of a pulsed Doppler ultrasound system that aims to detect alterations of diabetic foot cataloged as "high risk" associated with the processes that lead to occlusion of blood vessels.

In "Highly-integrated guidewire vascular ultrasound imaging system-on-a-chip" by J. Lim et al, 2018 IEEE Custom Integrated Circuits Conference (CICC), IEEE, 8 April 2018, pp. 1-4 (XP033339092), it is presented a proof-of-concept system-on-a-chip prototype for a guidewire ultrasound imaging system that includes interface electronics for ultrasound Tx/Rx and a quadrature sampler on-chip.

US 2016/097846 A1 relates to a system for ultrasound beamforming, including a sampled analog beamformer, an array of ultrasound transducers, and a high voltage amplifier coupled to the sampled analog beamformer and the array of ultrasound transducers. The sampled analog beamformer includes a sampled analog filter for filtering an incoming analog signal and adding a fractional delay, and for transmitting a filtered analog ultrasound signal. The array of ultrasound transducers further transmits the filtered analog ultrasound signal. The high voltage amplifier drives transducers in the array of ultrasound transducers.

### SUMMARY

Embodiments of the present disclosure are systems, devices, and methods for continuous wave (CW) Doppler ultrasound imaging. An ultrasound system can include a host, a probe, and a connecting cable between the host and the probe. The ultrasound imaging probe includes an array of ultrasound transducers which transmit ultrasound waves toward an anatomy and receive waves reflected from the anatomy. The received ultrasound waves may be used for CW Doppler imaging of velocities within the patient's anatomy. An example of such a velocity is the velocity of blood flow, e.g., between chambers of the heart (e.g., between an atrium and a ventricle). For CW Doppler imaging, some ultrasound imaging systems may transmit analog signals from each transducer element to the host via separate conductors requiring a connecting cable with many conductors. However, embodiments of the present disclosure perform some processing steps at the probe. According to the invention, analog signals may be summed and are mixed via in-phase and quadrature (I/Q) mixers within the probe. In some embodiments, CW Doppler signals may also be partially or completely beamformed and/or otherwise combined within the probe before or after I/Q mixing. Mixed CW Doppler signals are transmitted to the host via the connecting cable. Because signals are summed and mixed at the probe, the number of conductors in the connecting cable may be significantly reduced. In turn, the cost of the cable may also be significantly decreased. The cable may also become less cumbersome. While reducing the number of necessary conductors in the connecting cable, the present invention may additionally preserve the analog nature of CW Doppler signals sent to the processing system.

Embodiments of the present disclosure may additionally include processing components within the host system. The host system may receive digital B-mode ultrasound signals and generate an image of the patient's anatomy with various processing components or circuitry. The host system may receive analog CW signals and perform further processing to generate a graphical representation of velocity within the anatomy (e.g., blood flow).

In an exemplary aspect, an ultrasound probe in communication with an ultrasound system is provided. The ultrasound probe includes a transducer array configured to generate analog ultrasound signals; analog in-phase/quadrature (I/Q) mixers disposed within a housing of the ultrasound probe and in communication with the transducer array, wherein the analog I/Q mixers are configured to generate analog continuous wave (CW) Doppler signals based on the analog ultrasound signals; and a cable coupled to the housing, wherein the cable is configured to transmit the analog CW Doppler signals from the ultrasound probe to the ultrasound system.

In some aspects, the ultrasound probe further includes an analog-to-digital converter (ADC) disposed within the housing and in communication with the transducer array, wherein the ADC is configured to convert the analog ultrasound signals to digital ultrasound signals, and wherein the cable is configured to transmit the digital ultrasound signals to the ultrasound system. In some aspects, the ultrasound probe further includes at least one of a digital beamformer or a multiplexor in communication with the ADC. In some aspects, the cable comprises a first plurality of conductors configured to transmit the digital ultrasound signals. In some aspects, the cable comprises a second plurality of conductors configured to transmit the analog CW Doppler signals. In some aspects, the analog CW Doppler signals comprise I signals and Q signals, and the second plurality of conductors comprises: a first conductor configured to transmit the I signals; and a second conductor configured to transmit the Q signals. In some aspects, the ultrasound probe further includes a plurality of analog I/Q mixers disposed within the housing, wherein the plurality of analog I/Q mixers respectively correspond to a plurality of receive elements of the transducer array. In some aspects, the first conductor and the second conductor are electrically coupled to the plurality of analog I/Q mixers in parallel. In some aspects, respective outputs of the plurality of analog I/Q mixers are summed such that the first conductor and the second conductor transmit summed outputs of analog I/Q mixers. In some aspects, the ultrasound probe further includes a quadrature clock generator disposed within the housing and in communication with the analog I/Q mixers. In some aspects, the ultrasound probe further includes a plurality of conductors configured to transmit power, clock and control signals from the ultrasound system to the quadrature clock generator. In some aspects, the ultrasound probe further includes an analog beamformer disposed within the housing and in communication with the transducer array.

In an exemplary aspect, an apparatus is provided. The apparatus includes an ultrasound probe and an ultrasound system, wherein the ultrasound system is spaced from the ultrasound probe such that the cable extends between the ultrasound probe and the ultrasound system.

In some aspects, the ultrasound system comprises a processor circuit configured to: generate a graphical representation of a distribution of blood flow velocities based on the analog CW Doppler signals; and output the graphical representation to a display in communication with the processor circuit. In some aspects, the ultrasound probe is configured to convert the analog ultrasound signals to digital ultrasound signals, the cable is configured to transmit the digital ultrasound signals from the ultrasound probe to the ultrasound system, and the processor circuit is configured to: generate an ultrasound image of a heart based on the digital ultrasound signals; and output the ultrasound image to the display.

In an exemplary aspect, a method is provided. The method includes generating analog ultrasound signals with a transducer array of an ultrasound probe; generating analog CW Doppler signals based on the analog ultrasound signals with analog in-phase/quadrature (I/Q) mixers disposed within a housing of the ultrasound probe; transmitting the analog CW Doppler signals from the ultrasound probe to an ultrasound system spaced from the ultrasound probe by a cable coupled to the housing; generating, with a processor circuit of the ultrasound system, a graphical representation of blood flow velocities based on the analog CW Doppler signals; and outputting the graphical representation to a display in communication with the processor circuit.

Additional aspects, features, and advantages of the present disclosure will become apparent from the following detailed description.

### BRIEF DESCRIPTION OF THE DRAWINGS

Illustrative embodiments of the present disclosure will be described with reference to the accompanying drawings, of which:
Fig. 1 is a schematic diagram of an ultrasound imaging system, according to aspects of the present disclosure.
Fig. 2 is a schematic diagram of a processor circuit, according to aspects of the present disclosure.
Fig. 3 is a schematic diagram illustrating example circuitry of an ultrasound imaging probe, according to aspects of the present disclosure.
Fig. 4 is a schematic diagram illustrating example circuitry of an ultrasound imaging host system, according to aspects of the present disclosure.
Fig. 5A is a schematic diagram illustrating an example ultrasound transducer array, according to aspects of the present disclosure.
Fig. 5B is a schematic diagram illustrating an example ultrasound transducer array, according to aspects of the present disclosure.
Fig. 6 is a flow diagram of an ultrasound imaging method, according to aspects of the present disclosure.

### DETAILED DESCRIPTION

For the purposes of promoting an understanding of the principles of the present disclosure, reference will now be made to the embodiments illustrated in the drawings, and specific language will be used to describe the same. It is nevertheless understood that no limitation to the scope of the appended claims is intended. Any alterations and further modifications to the described devices, systems, and methods, and any further application of the principles of the present disclosure are fully contemplated and included within the present disclosure as would normally occur to one skilled in the art to which the disclosure relates. In particular, it is fully contemplated that the features, components, and/or steps described with respect to one embodiment may be combined with the features, components, and/or steps described with respect to other embodiments of the present disclosure. For the sake of brevity, however, the numerous iterations of these combinations will not be described separately.

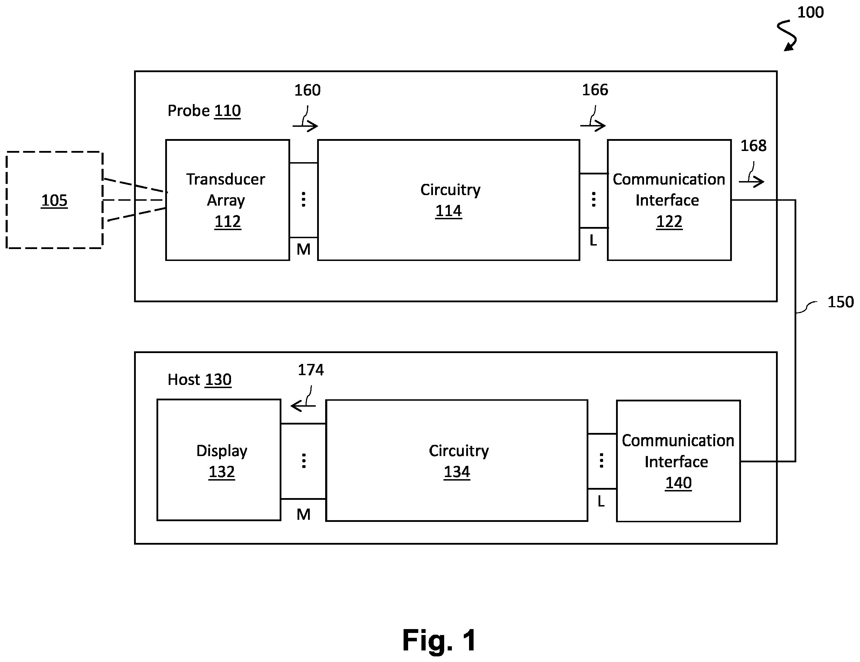

Fig. 1 is a schematic diagram of an ultrasound imaging system 100, according to aspects of the present disclosure. The system 100 may be used for scanning a region, area, or volume of a patient's body. The system 100 can be referred to as an apparatus in some instances. The system 100 includes an ultrasound imaging probe 110 in communication with a host 130 over a communication interface or link 150. At a high level, the probe 110 emits ultrasound waves towards an anatomical object 105 (e.g., a patient's body) and receives ultrasound echoes that are reflected from the object 105. The probe 110 transmits electrical signals representative of the received echoes over the link 150 to the host 130 for processing and image display. The probe 110 may be in any suitable form for imaging various body parts of a patient while positioned inside or outside of the patient's body. For example, the probe 110 may be in the form of a handheld ultrasound scanner or a patch-based ultrasound device. In some embodiments, the probe 110 can be an intra-body probe, such as a catheter, a transesophageal echocardiography (TEE) probe, and/or any other suitable an endo-cavity probe. The probe 110 includes a transducer array 112, various circuitry 114, and a communication interface 122.

The transducer array 112 emits ultrasound signals towards the object 105 and receives echo signals reflected from the object 105 back to the transducer array 112. The transducer array 112 may include acoustic elements arranged in a one-dimensional (1D) array, a 1.X-dimensional array, or a two-dimensional (2D) array. The acoustic elements may be referred to as transducer elements. Each transducer element can emit ultrasound waves towards the object 105 and can receive echoes as the ultrasound waves are reflected back from the object 105. For example, the transducer array 112 can include M transducer elements producing M analog ultrasound echo signals 160. In some embodiments, M can be about 2, 16, 64, 128, 192, 1000, 5000, 9000, and/or other suitable values both larger and smaller.

Circuitry 114 positioned within the probe 110 may be any of any suitable type and may serve several functions. For example, circuity 114 may include resistors, capacitors, transistors, inductors, relays, clocks, timers, or any other suitable electrical component that may be integrated in an integrated circuit. In addition, circuitry 114 may be configured to support analog signals and/or digital signals transmitted to or from the transducer array 112 and/or the probe 110. In some embodiments, circuitry 114 may include analog frontends (AFEs), analog-to-digital converters (ADCs), multiplexers (MUXs), and encoders, among other components. The circuitry 114 can include hardware components, software components, and/or a combination of hardware and software components.

The communication interface 122 is coupled to the circuitry 114 via L signal lines. In some embodiments, circuitry 114 may reduce the number of required lines from M signal lines to L signal lines. This may be accomplished by any suitable method using any suitable component. For example, MUXs, beamformers, or other components may be used to reduce the required signal lines M from the transducer array 112 to L signal lines 166. In the embodiment of Fig. 1, L is less than M. The communication interface 122 may be configured to transmit the L signals 166 to the host 130 via the communication link 150. The communication link 150 may include L data lanes for transferring the digital signals 168 to the host 130, as described in greater detail herein. The communication interface 122 may include hardware components, software components, or a combination of hardware components and software components configured to generate signals 168, carrying the information from the L signals 166, for transmission over the communication link 150. The signals 168 can be digital signals, analog signals, or a combination of digital signals and analog signals.

The host 130 may be any suitable computing and display device, such as a workstation, a personal computer (PC), a laptop, a tablet, a mobile phone, or a patient monitor. The host 130 can be referred to as an ultrasound system or an ultrasound host system. In some embodiments, the host 130 may be located on a moveable cart. At the host 130, the communication interface 140 may receive the digital and/or analog signals 168 from the communication link 150. The communication interface 140 may include hardware components, software components, or a combination of hardware components and software components. The communication interface may be substantially similar to the communication interface 122 in the probe 110.

Circuitry 134 positioned within host 130 may be any of any suitable type and may serve any suitable function. For example, circuity 134 may include resistors, capacitors, transistors, inductors, relays, clocks, timers, processing components, memory components, or any other suitable electrical component that may be integrated in an integrated circuit. In addition, circuitry 134 may be configured to support analog signals and/or digital signals transmitted to or from the probe 110. Circuitry 134 may be configured to process signals 168 received from the probe 110. For example, circuitry 134 may expand L signal lines received from the probe 110 to the original M signal lines corresponding to specific transducer elements or groups/patches of transducer elements within the transducer array 112. Circuitry 134 may additionally include a central processing unit (CPU), a digital signal processor (DSP), a graphical processing unit (GPU), an application-specific integrated circuit (ASIC), a controller, a field-programmable gate array (FPGA), another hardware device, a firmware device, or any combination thereof. Circuitry 134 may also be implemented as a combination of computing devices, e.g., a combination of a DSP and a microprocessor, a GPU and a microprocessor, a plurality of microprocessors, one or more microprocessors in conjunction with a DSP core, or any other such configuration. Circuitry 134 can be configured to generate image signals 174 for display to a user and/or perform image processing and image analysis for various diagnostic modalities or ultrasound types (B-mode, CW Doppler, etc.). For example, the circuitry 134 may be configured to process received digital ultrasound signals and generate an ultrasound image of the patient anatomy (e.g. a heart) and output the ultrasound image to the display 132. The circuitry 114 can include hardware components, software components, and/or a combination of hardware and software components.

The display unit 132 is coupled to circuitry 134. The display unit 132 may include a monitor, a touch-screen, or any suitable display. The display unit 132 is configured to display images and/or diagnostic results processed by circuitry 134. The host 130 may further include a keyboard, a mouse, a touchscreen or any suitable user-input components configured to receive user inputs for controlling the system 100

While Fig. 1 is described in the context of transmitting digital ultrasound echo signals from the probe 110 to the host 130 for display, the host 130 can generate signals for transmitting to the probe 110. For example, power signals, signals for controlling the probe 110 (e.g., exciting the transducer elements at the transducer array 112 to emit energy) can be transmitted by the host 130 to the probe 110 over the communication link 150.

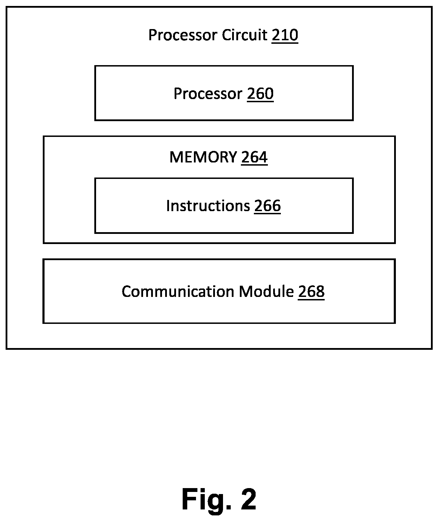

Fig. 2 is a schematic diagram of a processor circuit 210, according to aspects of the present disclosure. The processor circuit 210 may be implemented in the probe 110, the host system 130 of Fig. 1, or any other suitable location. One or more processor circuits 210 can be configured to perform the operations described herein. The processor circuit 210 can include additional circuitry or electronic components, such as those described herein. In an example, the processor circuit 210 may be in communication with the transducer array 112, circuitry 114, the communication interface 122, the communication interface 140, circuitry 134, and/or the display 132, as well as any other suitable component or circuit within the ultrasound system 100. In some embodiments, one or more components of the processor circuit 210 form at least a portion of the circuitry 114 or the circuitry 134. In some embodiments, one or more components of the circuitry 114 or the circuitry 134 form at least a portion of the processor circuit 210. In some instances, a distinct processor circuit 210 is implemented in the probe 110 and a distinct processor circuit 210 is implemented in the host 130. As shown, the processor circuit 210 may include a processor 260, a memory 264, and a communication module 268. These elements may be in direct or indirect communication with each other, for example via one or more buses.

The processor 260 may include a CPU, a GPU, a DSP, an application-specific integrated circuit (ASIC), a controller, an FPGA, another hardware device, a firmware device, or any combination thereof configured to perform the operations described herein. The processor 260 may also be implemented as a combination of computing devices, e.g., a combination of a DSP and a microprocessor, a plurality of microprocessors, one or more microprocessors in conjunction with a DSP core, or any other such configuration.

The memory 264 may include a cache memory (e.g., a cache memory of the processor 260), random access memory (RAM), magnetoresistive RAM (MRAM), read-only memory (ROM), programmable read-only memory (PROM), erasable programmable read only memory (EPROM), electrically erasable programmable read only memory (EEPROM), flash memory, solid state memory device, hard disk drives, other forms of volatile and non-volatile memory, or a combination of different types of memory. In an embodiment, the memory 264 includes a non-transitory computer-readable medium. The memory 264 may store instructions 266. The instructions 266 may include instructions that, when executed by the processor 260, cause the processor 260 to perform the operations described herein with reference to the probe 110 and/or the host 130 (Fig. 1). Instructions 266 may also be referred to as code. The terms "instructions" and "code" should be interpreted broadly to include any type of computer-readable statement(s). For example, the terms "instructions" and "code" may refer to one or more programs, routines, sub-routines, functions, procedures, etc. "Instructions" and "code" may include a single computer-readable statement or many computer-readable statements.

The communication module 268 can include any electronic circuitry and/or logic circuitry to facilitate direct or indirect communication of data between the processor circuit 210, the probe 110, and/or the display 132 and/or the display 266. In that regard, the communication module 268 can be an input/output (I/O) device. In some instances, the communication module 268 facilitates direct or indirect communication between various elements of the processor circuit 210 and/or the probe 110 (Fig. 1) and/or the host 130 (Fig. 1).

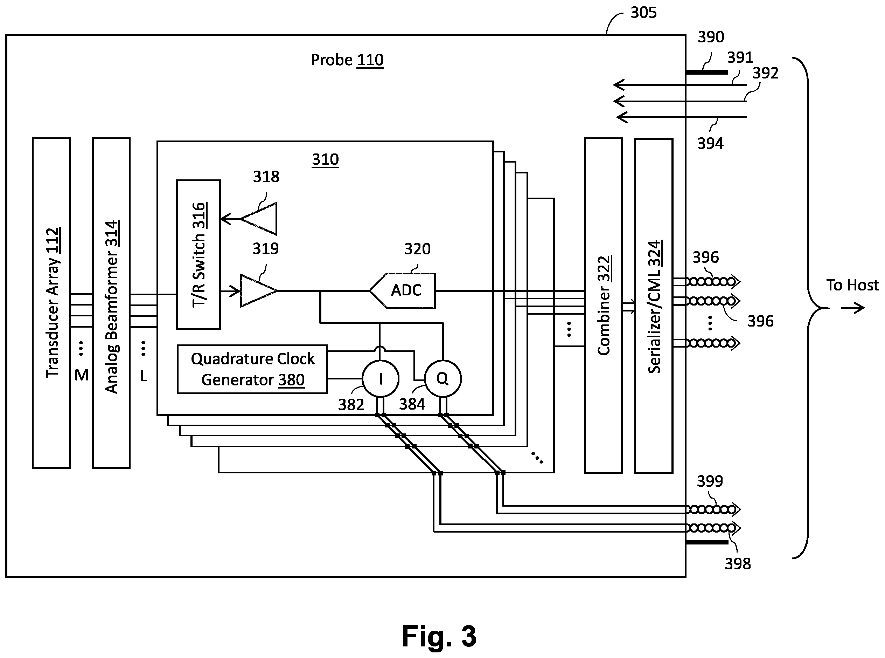

Fig. 3 is a schematic diagram illustrating example circuitry of an ultrasound imaging probe, according to aspects of the present disclosure. Fig. 3 provides a more detailed view of the probe 110 of the system 100 including transmission paths from the probe 110 to the host 130 and from the host 130 to the probe 110.

As shown in Fig. 3, the probe 110 further includes a housing 305, an optional analog beamformer 314, L circuitry blocks 310 (including, e.g., L transmit/receive switches (T/R switches) 316, transmit pulsers 318, preamplifiers 319, analog-to-digital converters (ADCs) 320, a quadrature clock generator 380, in-phase/quadrature mixers 382, 384), a combiner 322, and/or a serializer and high speed current mode logic (CML) 324. The probe 110 includes a plurality of circuitry blocks 310, each corresponding to a different signal channel associated with a group or sub-array of transducer elements of the transducer array 112. A connecting cable 390 is positioned between the probe 110 and the host 130 to establish signal communication. In that regard, the probe 110 and the host 130 can be spaced from one another. The connecting cable 390 extends between the probe 110 and the host 130. Circuitry within the host 130 can transmit signals to circuitry within the probe 110 via the cable 390. Circuitry within the probe 110 can transmit signals to circuitry within host 130 the via the cable 390. The cable 390 may include multiple signal lines including conductors, twisted pairs, coaxial cable, twin-axial cables, and/or any other suitable communication pathway for transferring data. The cable 390 can include a power conductor 394 for transmitting power from the host 130 to the probe 110. The cable 390 also includes a control signal line 392 and a clock line 391 for transmitting control and clock signals, respectively, from the host 130 to the probe 110. The cable 390 can also include one or more digital signal lines 396 for transmitting digital ultrasound signals from the probe 110 to the host 130, and I/Q signal lines 398, 399 for transmitting analog CW Doppler signals from the probe 110 to the host 130. The housing 305 may be any suitable enclosure of any suitable material and may house any or all of the components described herein. The cable 390 may be coupled to the housing 305.

The transmission path from the probe 110 to the host 130 may begin at the transducer array 112 shown in Fig. 3. The transducer array 112 may be coupled to the housing 305. The transducer array 112 may include M transducer elements. As previously stated, in some embodiments, M can be any suitable number and the transducer elements may be of any suitable type and in any suitable arrangement. The transducer array 112 generates analog ultrasound signals, or analog electrical signals representative of ultrasound echoes received at one or more transducer elements for any suitable imaging type (e.g., B-mode imaging, CW Doppler imaging, etc.). For CW Doppler imaging, one or more elements of the transducer array 112 are continuously emitting ultrasound energy simultaneously as one or more other elements of the transducer array 112 are continuously receiving ultrasound echoes (based on the emitted ultrasound energy). For example, half of the acoustic elements in the transducer array 112 can be transmitting while half of the acoustic elements in the transducer array 112 can be receiving.

The transducer array 112 may be in communication with the analog beamformer 314 via M signal lines. The analog beamformer 314 may be used to reduce the signal lines from the transducer array 112 to the rest of the circuitry 114 (Fig. 1) within the probe 110. For example, in some embodiments, the analog beamformer 314 may delay and sum the signals received from the transducer array 112 to create a smaller subset. The analog beamformer 314 may be a receive beamformer and/or a transmit beamformer. In embodiments in which the analog beamformer is a transmit beamformer, the analog beamformer 314 may include or be in communication with high voltage pulse generation circuitry. In other embodiments, for example, in embodiments where the transducer array 112 is a one-dimensional array of transducer elements or the number of transducer elements is otherwise reduced, the analog beamformer 314 may not be necessary or included within the probe 110. In some embodiments where the transducer array 112 is a one-dimensional array or the number of transducer elements is otherwise reduced, the analog beamformer 314 may still be included within the probe 110.

The analog beamformer 314 may be in communication with multiple T/R switches 316 via a reduced number of signal lines (e.g., L signal lines). The probe 110 can include one T/R switch 216 for every transducer element of the array 112 or for every group/patch of transducer elements. The T/R switches 316 may be configured to switch positions between different transmit and receive signal paths. For example, in a position for the transmit path, T/R switch 316 may transmit a high voltage activation signal from the pulser 318 to one or more elements of the transducer array 112 to activate one or more elements of the transducer array 112 to emit ultrasound energy. In receive mode, the T/R switch 316 may transmit receive signals corresponding to reflected waves received by the one or more transducer elements of the transducer array 112 to the preamplifier 319. The T/R switches 316 may be in communication with the host 130 via the control line 392 and may receive instructions regarding switching between various signal paths through the control line 392. The T/R switches 316 may also be in communication with the host 130 through any other suitable conductor or method.

The probe 110 may additionally include transmit pulsers 318. The transmit pulsers 318 may receive a command signal generated by the host 130. In response to the command signal, the transmit pulsers 318 generate electrical excitation pulses timed to cause the transducer array 112 to produce an acoustic transmit wave-front with any desired or specified focal characteristics.

The probe 110 may include L preamplifiers 319. The preamplifiers 319 may amplify signals received from the T/R switches 316 to improve the quality of received signals by, for example, reducing a noise floor. In some embodiments, the number of transmit pulsers 318 may be equal to the number of the transmit pulsers 318 and the number of T/R switches 316. For example, each T/R switch 316 may be configured to receive data from one pulser 318 and transmit data from the transducer array 112 to one preamplifier 319.

The receive signal path can be the same for CW Doppler imaging data and other imaging data (e.g., B-mode imaging data) from the transducer array to the preamplifiers 319. At the preamplifiers 319, the receive signal path diverges within the probe 110 to include different, parallel paths for CW Doppler imaging data and other imaging data. In the signal path for other imaging data, such as B-mode imaging data, each preamplifier 319 may be in communication with an ADC 320. The ADCs 320 may be configured to convert analog ultrasound echo signals into digital ultrasound echo signals. In that regard, the ultrasound probe 110 can generate digital ultrasound signals from the analog ultrasound signals and transmit the digital ultrasound signal to the host 130. For example, the ADCs 320 may receive analog ultrasound echo signals from the transducer array 112 via the T/R switches 316 and preamplifiers 319 and convert them into digital ultrasound echo signals. Digital ultrasound echo signals may include digital samples representing the waveforms of corresponding analog ultrasound echo signals. The ADCs 320 may employ a successive approximation ADC architecture to provide high-performance and lower-power consumption, and thus may keep total power dissipation of the probe 110 to be within a thermal budget of the probe 110. However, any suitable ADC architecture may be used for the ADCs 320.

Each ADC 320 may be in communication with the combiner 322. The combiner 322 is representative of circuitry that can reduce the total signal lines received from the ADCs 320 and reduce the number of required signal lines for transmitting data to the host 130. The combiner 322 may reduce the number of signal lines by any suitable method.
In some embodiments, the combiner 322 may include a summing node. The combiner 322, as well as any other suitable component or circuitry within the system 100 may include features similar to those described in US 2019/0227165 A1, titled "ULTRASOUND PROBE WITH MULTILINE DIGITAL MICROBEAMFORMER," and filed February 28, 2019 and/or WO 2019/158363 A1, titled "DIGITAL ULTRASOUND CABLE AND ASSOCIATED DEVICES, SYSTEMS, AND METHODS," and filed January 31, 2019. The combiner 322 may be a multiplexor and/or a digital beamformer. In some embodiments, the combiner 322 may be a multiplexor or may multiplex data received from the ADCs 320 into high-speed serial links and then send the data to the host 130 to be processed. In some embodiments, the combiner 322 may be a digital beamformer that performs a second stage of beamforming (delaying and summing of signals) after the first stage of beamforming is completed by the analog beamformer 214. The combiner 322 may be in communication with the serializer and high speed current mode logic (CML) 324. The serializer/CML 324 may rearrange lines received from the combiner 322 and/or the ADC's 320 into a high rate serial data stream. In some embodiments, the serializer/CML 324 may run at a higher data rate than other circuitry within the probe 110. For example, the serial data stream may run at 160 MHz whereas other circuitry within the ultrasound signal path may run at 20 MHz. The serializer/CML 324 may operate in a similar manner to the serializer disclosed in WO 2019/158363 A1, titled "DIGITAL ULTRASOUND CABLE AND ASSOCIATED DEVICES, SYSTEMS, AND METHODS," and filed January 31, 2019. Accordingly, in one of the signals paths of the probe 110, digital ultrasound data (e.g., B-mode data) can be transmitted from the probe 110 to the host 130 via the conductors 396. The conductors 396 may be a twisted pairs of conductors, coaxial cables, twin-axial cables, and/or any other suitable signal pathway. In general, one or a plurality of conductors can transmit the digital ultrasound signals from the probe 110 to the host 130.

In the parallel CW Doppler imaging path, the ultrasound probe 110 generates analog CW Doppler signals from the analog ultrasound signals and transmit the analog CW Doppler signals to the host 130. The circuitry block 310 of the ultrasound probe 110 includes quadrature clock generators 380. Each quadrature clock generator 380 may be in communication with analog I/Q mixers 382, 384. The analog I/Q mixers 382, 384 may be disposed within the housing 305 and in communication with the transducer array 112. The I/Q mixers 382, 384 generate analog CW Doppler signals within the probe 110, which are then transmitted to the host 130. In particular, the I/Q mixers 382, 384 generate analog baseband quadrature outputs. Fig. 3 depicts the I mixers 382, the Q mixers 384, and the quadrature clock generators 380 positioned within the probe 110. For each circuitry block 310 or ultrasound channel, the respective I/Q mixers generate CW Doppler signals for the ultrasound data corresponding to the associated group or sub-array of acoustic element. The CW Doppler signals from each circuitry block 310 may be converted to baseband and summed prior to being transmitted to the host 130. This reduces the number of conductors required to transmit the CW Doppler signal data, compared to if a different conductor was required for each ultrasound channel or circuitry block 310. In some embodiments, the analog CW Doppler signals are transmitted from the probe 110 to the host 130 via 2 signal lines: an I signal line 398 corresponding to the output of summed I mixers 382 and a Q signal line 399 corresponding to the output of summed Q mixers 384. Stated differently, the outputs of the I mixers 382 and the Q mixers 384, respectively, are electrically connected in parallel so as to create two signal lines: the I signal line 398 and the Q signal line 399. In this way, the I signal line 398 carries the summed outputs of the I mixers 382 within the housing 305 and the Q signal line 399 carried the summed outputs of the Q mixers 384 within the housing 305.

The quadrature clock generator 380 may include two outputs, one of which is in communication with an I mixer 382 and the other is in communication with a Q mixer 384. The quadrature clock generator 380 may produce a delay difference between the output of the I mixer 382 and the output of the Q mixer 384. The delay difference of the Q mixer 384 may be equal to one-quarter of a clock phase from the clock signal of the I mixer 382 such that the I mixer 382 produces an in-phase signal and the Q mixer 384 produces a quadrature signal. In some embodiments, the quadrature clock generator 380 may generate a signal that is substantially similar to a square wave. The quadrature clock generator 380 may include any suitable electrical components to generate the phase difference between the I mixer 382 and the Q mixer 384. For example, the quadrature clock generator 380 may include one or more edge triggered D flip-flops or any other suitable flip-flops, inverters such as tri-state inverters, or any other suitable electrical component. The quadrature clock generator 380 receives power, clock and control signals from the host 130 through connections 394, 391, and 392, respectively.

The I mixers 382 are positioned within the probe 110 with one I mixer 382 for each circuitry block 310. In some embodiments, the I mixers 382 may be multiplying mixers. In other embodiments, the I mixers 382 may be any suitable mixers of any particular type. An I mixer 382 may include two inputs. One input may be in communication with the output of the preamplifier 319 within a circuitry block 310 and may receive ultrasound signals. The other input may be in communication with the output of the quadrature clock generator 380. The I mixer 382 may multiply the signal received by the transducer array 112 with the signal received from the quadrature clock generator 380 and output the result. The output signal from the I mixer 382 may therefore correspond to a sum and a difference of the two input signals.

Similar to the I mixer 382, the Q mixers 384 are also positioned within the probe 110 with one Q mixer 384 for each circuitry block 310. The Q mixer 384 may also be a multiplying mixer or any other suitable type of mixer. The Q mixer 384 may be substantially similar to the I mixer 382. However, the Q mixer 384 may differ from the I mixer 382 in that it receives a phase shifted signal from the quadrature clock generator 380. However, like the I mixer 382, the Q mixer 384 may also multiply two inputs, one corresponding to the output of the preamplifier 319 (the electrical signals generated by the transducer array 112 in response to the received echoes) and the other in communication with the output of the quadrature clock generator 380. Like the I mixer 382, the Q mixer 384 may also reduce the CW Doppler signal content received from the transducer array 112 to baseband prior to transmitting to the host 130.

As previously stated, multiple I mixers 382 and Q mixers 384 are positioned within the probe 110. In some embodiments, an I mixer 382 may be present on each circuitry block 310 and a Q mixer 384 may be present on each circuitry block 310. In some embodiments, the outputs from each I mixer 382 and each Q mixer 384 may be transmitted via a twisted pair, a coaxial cable, a twin-axial cable, and/or other suitable conductor from the probe 110 to the host 130. In other embodiments, and as shown in Fig. 3, the outputs of all I mixers 382 within the probe 110 may be summed together into a single twisted pair 398, coaxial cable, twin-axial cable, or other wire type, corresponding to the I mixer output signal before transmitting to the host 130. In general, one or a plurality of conductors can transmit the I signal from the probe 110 to the host 130. Similarly, the outputs of all the Q mixers 384 within the probe 110 may be summed together into a single twisted pair 399, coaxial cable, twin-axial cable, or other wire type, corresponding to the Q mixer output signal before transmitting to the host 130. In general, one or a plurality of conductors can transmit the Q signal from the probe 110 to the host 130. The I signal line 398 may be a twisted pair or any other suitable conductor, such as a coaxial cable or twin-axial cable. The Q signal line 399 may be substantially similar to the I signal line 398. Because the I/Q outputs, respectively, are connected in parallel, only one signal pathway (e.g., twisted pair, coaxial cable, twin-axial cable, and/or any suitable conductor) is needed to carry the summed I signal on the I signal line 398 and only one signal pathway (e.g., twisted pair, coaxial cable, twin-axial cable, and/or any suitable conductor) is needed to carry the summed Q signal on the Q signal line 399. Advantageously, this reduces the quantity of analog CW Doppler signal carrying lines in the cable 390. For example, a CW Doppler signal line for each ultrasound channel (each circuit block 310) is advantageously avoided, as this would add undesired bulk and expense for the cable 390.

The probe 110 transmits digital ultrasound data via conductors 396 and analog CW Doppler signals via the conductors 398 and 399. An advantage of the present disclosure includes maintaining the analog nature of the CW Doppler signals transmitted from the probe 110 to the host 130. Transmitting analog CW Doppler signals to the host 130 may prevent artefacts in the data inherent in some digital conversion processes. As a result, analog CW Doppler signals may retain original signal quality and lead to better quality images and/or fluid velocity measurements. In addition, analog CW Doppler signals may be processed using similar components or techniques within the host as extant systems, which results in reduced cost of implementation.

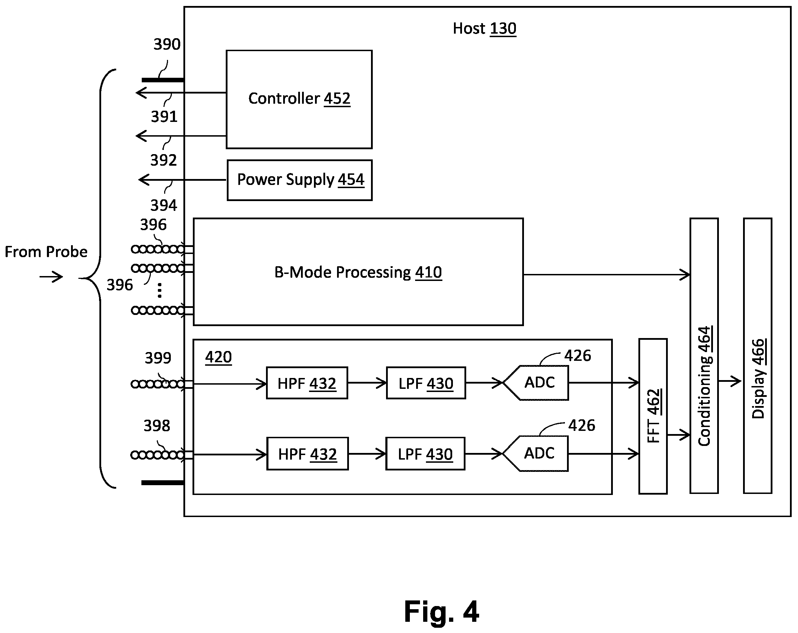

Fig. 4 is a schematic diagram illustrating example circuitry of an ultrasound imaging host system 130, according to aspects of the present disclosure. Fig. 4 provides a more detailed view of the host 130 of the system 100 including transmission paths from the probe 110 to the host 130 and from the host 130 to the probe 110. As shown in Fig. 4, the host 130 may include a controller 452, power supply 454, a B-mode processing circuit block 410, a CW Doppler processing circuit block 420, a fast Fourier transform (FFT) processing block 462, a conditioning block 464, and a display 466.

The controller 452 within the host 130 may control the operations of any number of components within the probe 110 and/or the host 130. For example, the controller 452 may control the combiner 322 and/or the serializer/CML 324 (Fig. 3). The controller 452 may generate control data for operating the transducer elements at the transducer array 112, for example, for ultrasound wave emissions. The controller 452 may further control the analog beamformer 314, the T/R switches 316, the pulsers 318, the preamplifiers 319, and the quadrature clock generators 380 (Fig. 3). The controller may also be in communication with components within the B-mode processing circuitry block 410 and/or the CW Doppler processing circuitry block 420, including an encoder, serializing and/or de-serializing components, transmitters, or any other suitable components within the host 130. In some embodiments, the controller 452 could be a processor circuit or could be a part of a processor circuit. The controller 452 could be part of the processor circuit 210 shown in Fig. 2. The control line 392 may be in communication with the controller 452 within the host 130 and may provide signals for controlling components within the probe 110. In some embodiments, the control line 392 and/or the clock line 391 may be a twisted pair. In other embodiments, the control line 392 and/or the clock line 391 may be a conductor, coaxial cable, twin-axial cable, and/o any other suitable signal communication pathway for transmitting data signals. In some embodiments, data transmitted via the control line 392 may be 800Mbs data, or data of any suitable frequency or type. Signals transmitted from the host 130 to the probe 110 via control line 392 and/or clock line 391 may be analog or digital signals. When digital command signals are transmitted, the data may be transmitted via the data line 292 at any suitable bit rate, such as between 400 Mbit/s and 8Gbit/s, including values such as 2.4Gbit/s and/or other suitable values both larger and smaller.

The power supply 454 may provide power to the host 130 and to the probe 110 (e.g., any suitable components within the probe 110 or the host 130). The power line 394 may be in communication with a power supply 454 within the host 130 or at any suitable location in relation to other components. The power line 394 may provide electrical power to various components within the probe 110.

The processing circuit block 410 may receive digital ultrasound signals via the signal lines 396. The processing circuit block 410 may include any suitable components used for processing digital ultrasound data, generating an ultrasound image, and outputting display data for display to a user of the ultrasound system 100 on the display 466. In that regard, the processing circuit block 410 can be implemented as hardware components, software components, and/or a combination of hardware and software components. For example, the circuit block 410 may include encoders, serializing components, de-serializing components, transmitters, decoders, multiplexors, de-multiplexors, beamformers, or any other suitable components. The circuit block 410 may also include signal processing components, scan converter components, controllers, or other components. The circuit block 410 may be configured to display to a user a depiction of tissues, organs, or other structures within a patient anatomy. The processing circuit block 410 can be used for processing digital B-mode ultrasound imaging signals. In other embodiments, the processing circuit block 410 is representative of the circuitry used for may be processing circuitry for any suitable ultrasound imaging type (e.g., B-mode imaging, 3D/4D imaging, M-mode imaging, color flow Doppler imaging, or any other suitable form or type of ultrasound imaging).

The CW Doppler processing circuit block 420 receives analog CW Doppler signals via the signal lines 398, 399. The CW Doppler processing circuit block 420 may include a plurality of high pass filters (HPFs) 432, anti-aliasing low pass filters (LPFs) 430, and analog-to-digital converters (ADCs) 426. In that regard, the CW Doppler processing circuit block 420 can include HPF 432, LPF 430, and ADC 426 for each of the I signal pathway and the Q signal pathway. The HPFs 432 can be referred to as wall filters. The HPFs 432 and the LPFs 430 can be analog components.

In the illustrated embodiment, the HPFs or wall filters 432 operate on the analog CW Doppler signals. The HPFs 432 may be positioned either within the probe 110 or the host 130 as shown in Fig. 4. One HPF 432 may be in communication with the summed output of I mixers 382 via I signal line 398 and an additional HPF 432 may be in communication with the summed output of Q mixers 384 via Q signal line 399. The wall filters 432 may include additional circuitry within the host 130. The wall filters 432 may also include op amps. The HPFs 432 may filter out low Doppler signals corresponding to arterial walls or any other static tissue within a patient. The HPF 432 may additionally filter high amplitude low frequency content from movement within a patient from, for example, heart beats, general patient or probe movement, or other movement. In some embodiments, the HPF 432 may be an aggressive filter.

After signals are processed through the HPF 432, a LPF 430 may be used to remove high frequency energy that would otherwise be aliased into the pass band by the sampling function of analog to digital convertor 426. In that regard, CW Doppler ultrasound signals are obtained when a high frequency ultrasound energy is emitted by the transducer array 112 and propagates into the anatomy of a patient. Ultrasound echoes, based on the emitted ultrasound energy, that are received at the transducer array 112 may be of a slightly higher or lower frequency compared to the emitted ultrasound energy and correspond to moving liquids such as blood flow. The relevant information for CW Doppler imaging to be extracted from the ultrasound signal is then the difference between the transmitted and received ultrasound energy. The output of the I mixer 382 corresponding to the sum and difference between the inputs of the I mixer 382 may then be filtered by LPF 430 to remove the high frequency content corresponding to the sum, leaving only the lower audio-level frequencies or baseband frequencies for additional processing. This filtering may be performed within the probe 110, within the circuitry block 310, or within the host 130 after signals have been transmitted to the host 130. The output of the Q mixer 384 may also be filtered by LPF 430 like the I mixer 382. This filtering may be performed within the probe 110, within circuitry block 310, or within the host 130 after signals have been transmitted to the host 130.

Following the LPF 430, one or more analog-to-digital converters (ADCs) 426 may be utilized to convert analog CW Doppler signals to digital CW Doppler signals. In some embodiments, the ADCs 426 could be positioned after the LPF 430 and before the FFT 462. However, in other embodiments, the ADCs 426 may be positioned at any other location within the host 130 along the signal chain. For example, the ADCs 426 could be positioned before the HPFs 432, between the HPFs 432 and the LPF 430 or at any other suitable location. All circuitry after the position of the ADCs 426 may therefore be of a digital implementation or could be implemented via software and/or hardware circuitry, while processing circuits before the positions of the ADC's 426 in the signal chain may be of an analog implementation. Thus, in the illustrated embodiment of Fig. 4, the HPF 432 and the LPF 430 can be analog components. In other embodiment, the HPF 432 and the LPF 430 could be digital components.

The fast Fourier transform (FFT) 462 may be applied to the signal data output from the CW Doppler processing circuitry block 420 to create a Doppler spectrum associated with velocity of movement within a patient. Following the FFT 462, the signal data may be further processed at conditioning 464 and then output for display to a user via display 466. In that regard, for CW Doppler imaging, a graphical representation of distribution of blood flow velocities is output via the display 466. It is fully contemplated that any suitable form of data processing may be applied to the signal data at this or any stage in the circuitry of the present invention. For example, the host 130 may apply additional data processing techniques to enhance the quality of the signal data, identify or emphasize various characteristics or aspects of the signal data, etc.

Fig. 4 additionally depicts the connecting cable 390 positioned between the probe 110 and the host 130. The cable 390 may include multiple signal lines including conductors, twisted pairs, coaxial cables, twin-axial cables, or any other suitable communication pathway of transferring data. In some embodiments, the cable can be replaced with an optical or a wireless interface. For example, the cable 390 may include the control line 392, the power line 394, the clock line 391, and multiple signal lines 396 previously discussed. Multiple signal lines 396 may correspond to a reduced number of signal lines output from the combiner 322 and/or the serializer/CML 324. In some embodiments, the signal lines 396 may include only a single signal line. In other embodiments, the signal lines 396 may include more than one. The cable 390, and any corresponding cables enclosed within the cable 390 such as the control line 392, the clock line 391, the signal lines 396, the power line 394, and/or the I mixer line 398 and the Q mixer line 399 may be of any suitable length and/or may be a flexible elongate member. For example, the cable 390 and all associated conductors may be 1 meter, 2 meters, 3 meters in length, or more, or any suitable length therebetween.

In some embodiments, the B processing circuitry block 410 and the CW Doppler processing circuitry block 420 are composed of separate components and separate signal paths. In some embodiments, components or sets of components within the processing circuitry block 410 may be shared with the CW Doppler processing circuitry block 420 or any other circuitry or components within the host 130.

In some embodiments, the B-mode processing circuitry block 410 could be a processor circuit or could be a part of a processor circuit. The B-mode processing circuitry block 410 could be part of the processor circuit 210 shown in Fig. 2. The B-mode processing circuitry block 410 could include any sort of suitable processing circuit including one or more of any components of the processor circuit 210. Similarly, the CW Doppler processing circuitry block 420 could be a processor circuit or could be a part of a processor circuit or could be part of the processor circuit 210 shown in Fig. 2. The CW Doppler processing circuitry block 420 could also include any sort of suitable processing circuit including one or more of any component of processor circuit 210.

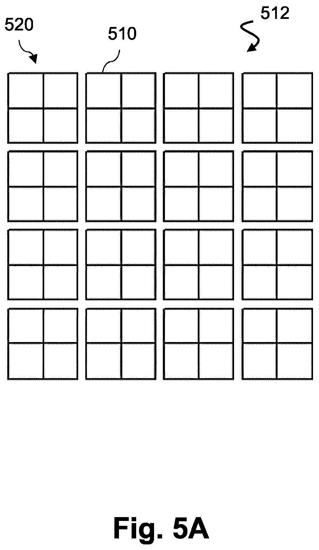

Fig. 5A is a schematic diagram illustrating an example ultrasound transducer array 512, according to aspects of the present disclosure. The ultrasound transducer array 512 includes multiple ultrasound transducers 510 arranged into sub-arrays 520.

The transducer array 512 shown in Fig. 5A may be a 1.X-dimensional or two-dimensional matrix of ultrasound elements 510. The transducer array 512 may be substantially similar to the transducer array 112 of Fig. 1 and/or Fig. 3. In other embodiments, the transducer array 512 may also be a 1-dimensional linear array, or any other suitable type of array. As previously mentioned in regards to the transducer array 112, the transducer array 512 may include any suitable number of transducer elements 510. The transducer elements 510 may be arranged within the transducer array 512 in multiple sub-arrays 520. The sub-arrays 520 may additionally be referred to as groups or patches, among other suitable terms. Each sub-array 520 may include four transducer elements 510, or any other suitable number of transducer elements 510. For example, the sub-array 520 may comprise 2, 4, 6, 8, 10, 12, or more transducer elements 510 as well as any suitable number therebetween. In addition, in some embodiments, each sub-array 520 need not include the same number of transducer elements 510, but each could vary according to any suitable arrangement or pattern. It is noted that the spacing between the sub-arrays 520 shown in Fig. 5A does not necessarily indicate physical spacing or separation within the array 512. For example, each of the transducer elements 510 in the array can have the same space with each adjacent element (whether or not that element is part of the same sub-array 520). Rather, the spacing shown in Fig. 5A can be illustrative of the sub-array groupings.

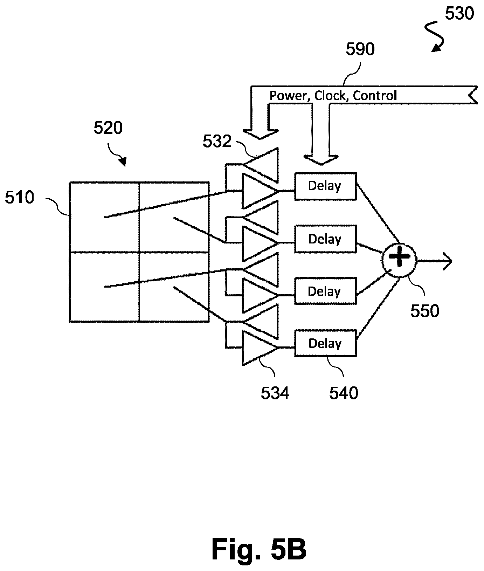

Fig. 5B is a schematic diagram illustrating example circuitry of an analog beamformer 530, according to aspects of the present disclosure. The analog beamformer 530 may be substantially similar to the analog beamformer 314 of Fig. 3. Fig. 5B provides a more detailed view of the analog beamformer 530, which may be implemented within the ultrasound probe. The analog beamformer 530 includes multiple transmit pulsers 532, preamplifiers 534, delay circuits 540, a summation component 550, and conductors 590 providing power, clock, and/or control signals to any of these components. Fig. 5B additionally depicts one sub-array 520 including multiple ultrasound transducer elements 510. The sub-array 520 shown in Fig. 5B may be one of the sub-arrays 520 shown in Fig. 5A or may be a different sub-array.

The transmit pulsers 532 may be substantially similar to the pulsers 318 of Fig. 3. Specifically, the transmit pulsers 532 may receive command signals from the host and in response to these command signals, transmit high-voltage pulses to activate the ultrasound elements 510 to emit ultrasound energy that propagates into a patient's anatomy. Each ultrasound element 510 may therefore correspond to and/or be in communication with a transmit pulser 532.

Multiple preamplifiers 534 are additionally depicted in Fig. 5B. The preamplifiers 534 may be substantially similar to the preamplifiers 319 of Fig. 3. The preamplifiers 534 may amplify signals received from the ultrasound elements 510 to improve the quality of received signals by, for example, reducing a noise floor.

Multiple delay circuits 540 may be in communication with the preamplifiers 534 within the analog beamformer 530. The delay circuits 540 may be of any suitable type. For example, the delay circuits 540 may include analog delay circuitry for the analog beamformer 530. The delay circuits 540 may apply a delay profile to signals received from the ultrasound transducers 510 so as to perform beamforming in relation to all elements within a sub-array 520 or partial beamforming. Such delay profiles may be provided to the delay circuits 540 via any suitable method. For example, in some embodiments, a conductor corresponding to control or clock data within the conductors 590 may be in communication with the delay circuits 540 and may dictate delay profiles for the delay circuits 540.

Fig. 5B additionally depicts a summation component 550. The summation component 550 may be an analog adder circuit, summing mixer, or any suitable electronic component for summing signals. The summation component 550 is in communication with the respective outputs of the delay circuits 540. In such a configuration, the signals output from each delay circuit 540 may be summed in an analog fashion. In other embodiments, the summation component 550 may comprise any suitable circuitry or configuration to otherwise combine signals from the outputs of the delay circuits 540. The output of the summation component 550 may then be in communication with one or more T/R switches 316 from Fig. 3 and the signals combined by the analog beamformer 530 may be further processed and/or combined within the probe 110 and/or the host 130 as has been described or in any other suitable way.

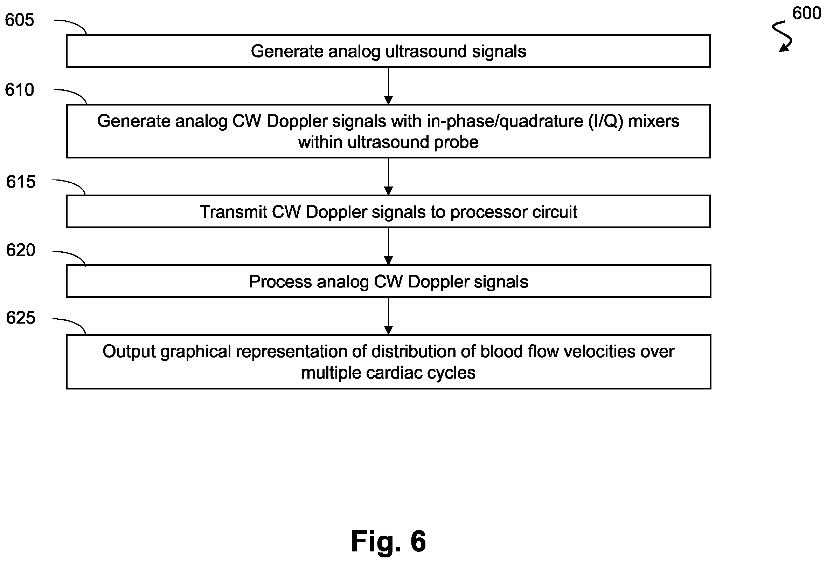

Fig. 6 is a flow diagram of an ultrasound imaging method 600, according to aspects of the present disclosure. As illustrated, the method 600 includes a number of enumerated steps, but embodiments of the method 600 may include additional steps before, after, or in between the enumerated steps. In some embodiments, one or more of the enumerated steps may be performed concurrently. The steps of the method 600 can be carried out by any suitable component within ultrasound imaging system 100 and all steps need not be carried out by the same component. In some embodiments, one or more steps of the method 600 can be performed by, or at the direction of, a processor circuit of the ultrasound imaging system 100, including, e.g., the processor 260 (Fig. 2) or any other component.

At step 605, the method 600 includes generating analog ultrasound signals. Command signals may be generated at the host 130 and transmitted to the probe 110 via the signal line 392. The pulsers 318 may consequently generate a signal to excite the transducer elements of the transducer array 112 to generate ultrasound waves (Fig. 3). The transducer array 112 may then also receive echo signals reflected from features in the patient's anatomy and generate analog electrical signals representative of the ultrasound echoes. The generated analog ultrasound signals may then be transmitted to the circuitry block(s) 310 (Fig. 3).

At step 610, the method 600 includes generating, based on the analog ultrasound signals, analog continuous wave (CW) Doppler signals with in-phase/quadrature (I/Q) mixers within the ultrasound probe 110. The CW Doppler signals may be generated using multiple I mixers 382 and Q mixers 384 (Fig. 3). The I mixers 382 and Q mixers 384 may include multiplying mixers or any other suitable type of mixer. The probe 110 may include one or more quadrature clock generators 380 (Fig. 3) which may provide an additional input to the I mixers 382 and the Q mixers 384 to reduce analog ultrasound signals to baseband frequencies and create an appropriate phase shift between the output of the I mixers 382 and the output of the Q mixers 384.

At step 615, the method 600 includes transmitting the analog CW Doppler signals to a processor circuit within the host 130. The CW Doppler signals may be transmitted to the host 130 via the cable 390, conductors, twisted pairs, coaxial cables, twin-axial cables, or any other appropriate signal line within the cable 390, or via any suitable method.

At step 620, the method 600 includes processing the analog CW Doppler signals. Processing of the analog CW Doppler signals may include any suitable data processing component or procedure, including filtering via low pass filters, high pass filters or any suitable type of filter. Data processing may additional include windowing, summations, averaging, smoothing, transformations from one domain to another, such as with fast Fourier transforms, and any other suitable conditioning to improve the overall data quality, clarity, or presentation. Signal processing may additionally include converting analog CW Doppler signals to digital CW Doppler signals. In such an embodiment, signal processing may also be performed digitally, via a standard personal computer and/or processor, in software form, or with hardware, such as physical circuitry within the host 130 or via any other suitable method or form.

At step 625, method 600 includes generating graphical representations of blood flow velocities over one or multiple cardiac cycles. Graphical representations may include any suitable data presentation. For example, graphical representations may include simple lists of data including time, velocities, dimensions, or data relating to the location of an imaged object within a patient's anatomy. Graphical representations may additionally include a Doppler spectrum or other applicable spectrum, plot, or other graphical representation. Graphical representations may also include any suitable plots, pictures, or depictions which may convey to a user information regarding the health or physical state of a patient. Graphical representations of the distribution of blood flow velocities, or other fluid velocities, may be output to a display 132 (Fig. 1) in communication with the processor circuit 134 (Fig. 1) or any other suitable processor described herein.

Persons skilled in the art will recognize that the apparatus, systems, and methods described above can be modified in various ways. Accordingly, persons of ordinary skill in the art will appreciate that the embodiments encompassed by the present disclosure are not limited to the particular exemplary embodiments described above. In that regard, although illustrative embodiments have been shown and described, a wide range of modification, change, and substitution is contemplated in the foregoing disclosure. It is understood that such variations may be made to the foregoing without departing from the scope defined by the appended claims. Accordingly, it is appropriate that the appended claims be construed broadly and in a manner consistent with the present disclosure.

## Claims

1. An ultrasound probe (110) adapted to communicate with an ultrasound system (130), the ultrasound probe comprising:
a transducer array (112) configured to generate analog ultrasound signals (160);
analog in-phase/quadrature (I/Q) mixers (382, 384) disposed within a housing (305) of the ultrasound probe and in communication with the transducer array, wherein the analog I/Q mixers are configured to generate analog continuous wave (CW) Doppler signals based on the analog ultrasound signals; and
a cable (390) coupled to the housing, wherein the cable is configured to transmit the analog CW Doppler signals from the ultrasound probe to the ultrasound system.

2. The ultrasound probe (110) of claim 1, further comprising:
an analog-to-digital converter (ADC) (320) disposed within the housing (305) and in communication with the transducer array (112), wherein the ADC is configured to convert the analog ultrasound signals (160) to digital ultrasound signals (168), and wherein the cable (390) is configured to transmit the digital ultrasound signals to the ultrasound system (130).

3. The ultrasound probe (110) of claim 2, further comprising:
at least one of a digital beamformer or a multiplexor in communication with the ADC (320).

4. The ultrasound probe (110) of claim 2, wherein the cable (390) comprises a first plurality of conductors (396) configured to transmit the digital ultrasound signals.

5. The ultrasound probe (110) of claim 4, wherein the cable (390) comprises a second plurality of conductors (398, 399) configured to transmit the analog CW Doppler signals.

6. The ultrasound probe (110) of claim 5,
wherein the analog CW Doppler signals comprise I signals and Q signals, and
wherein the second plurality of conductors comprises:
a first conductor (398) configured to transmit the I signals; and
a second conductor (399) configured to transmit the Q signals.

7. The ultrasound probe (110) of claim 6, further comprising:
a plurality of analog I/Q mixers (382, 384) disposed within the housing (305), wherein the plurality of analog I/Q mixers respectively correspond to a plurality of receive elements of the transducer array (112).

8. The ultrasound probe (110) of claim 7, wherein either:
the first conductor (398) and the second conductor (399) are electrically coupled to the plurality of analog I/Q mixers (382, 384) in parallel;
or
respective outputs of the plurality of analog I/Q mixers (382, 384) are summed such that the first conductor (398) and the second conductor (399) transmit summed outputs of analog I/Q mixers.

9. The ultrasound probe (110) of claim 1, further comprising:
a quadrature clock generator (380) disposed within the housing (305) and in communication with the analog I/Q mixers (382, 384).

10. The ultrasound probe (110) of claim 9, wherein the cable (390) comprises a plurality of conductors (394, 391, 392) configured to transmit power, clock and control signals from the ultrasound system (130) to the quadrature clock generator (380).

11. The ultrasound probe (110) of claim 1, further comprising:
an analog beamformer (314) disposed within the housing (305) and in communication with the transducer array (112).

12. An apparatus (100), comprising:
the ultrasound probe (110) of claim 1; and
the ultrasound system (130), wherein the ultrasound system is spaced from the ultrasound probe such that the cable (390) extends between the ultrasound probe and the ultrasound system.

13. The apparatus (100) of claim 12, wherein the ultrasound system (130) comprises a processor circuit (210) configured to:
generate a graphical representation of a distribution of blood flow velocities based on the analog CW Doppler signals; and
output the graphical representation to a display (466) in communication with the processor circuit.

14. The apparatus (100) of claim 13,
wherein the ultrasound probe (110) is configured to convert the analog ultrasound signals (160) to digital ultrasound signals (168),
wherein the cable (390) is configured to transmit the digital ultrasound signals from the ultrasound probe to the ultrasound system, and wherein the processor circuit (210) is configured to:
generate an ultrasound image of a heart based on the digital ultrasound signals; and
output the ultrasound image to the display (466).

15. A method (600), comprising:
generating (605) analog ultrasound signals with a transducer array of an ultrasound probe;
generating (610) analog CW Doppler signals based on the analog ultrasound signals with analog in-phase/quadrature (I/Q) mixers disposed within a housing of the ultrasound probe;
transmitting (615) the analog CW Doppler signals from the ultrasound probe to an ultrasound system spaced from the ultrasound probe by a cable coupled to the housing;
generating (620), with a processor circuit of the ultrasound system, a graphical representation of blood flow velocities based on the analog CW Doppler signals; and
outputting (625) the graphical representation to a display in communication with the processor circuit.

## Patentansprüche

1. Ultraschallsonde (110), die zur Kommunikation mit einem Ultraschallsystem (130) angepasst ist, wobei die Ultraschallsonde Folgendes umfasst:
ein Wandlerarray (112), das zum Erzeugen analoger Ultraschallsignale (160) konfiguriert ist;
analoge In-Phase-&-Quadrature- (I/Q)- Mischer (382, 384), die in einem Gehäuse (305) der Ultraschallsonde angeordnet sind und mit dem Wandlerarray in Kommunikation stehen, wobei die analogen I/Q-Mischer dazu konfiguriert sind, analoge Dauerstrich-(CW)- Dopplersignale auf der Grundlage der analogen Ultraschallsignale zu erzeugen; und
ein mit dem Gehäuse gekoppeltes Kabel (390), wobei das Kabel dazu konfiguriert ist, die analogen CW-Dopplersignale von der Ultraschallsonde an das Ultraschallsystem zu übertragen.

2. Ultraschallsonde (110) nach Anspruch 1, weiter umfassend:
einen Analog-Digital-Wandler (ADC) (320), der im Gehäuse (305) angeordnet ist und mit der Wandleranordnung (112) in Kommunikation steht, wobei der ADC dazu konfiguriert ist, die analogen Ultraschallsignale (160) in digitale Ultraschallsignale (168) umzuwandeln, und wobei das Kabel (390) dazu konfiguriert ist, die digitalen Ultraschallsignale an das Ultraschallsystem (130) zu übertragen.

3. Ultraschallsonde (110) nach Anspruch 2, weiter umfassend:
mindestens einen digitalen Strahlformer oder einen Multiplexer in Kommunikation mit dem ADC (320).

4. Ultraschallsonde (110) nach Anspruch 2, wobei das Kabel (390) eine erste Vielzahl von Leitern (396) umfasst, die zum Übertragen der digitalen Ultraschallsignale konfiguriert sind.

5. Ultraschallsonde (110) nach Anspruch 4, wobei das Kabel (390) eine zweite Vielzahl von Leitern (398, 399) umfasst, die zum Übertragen der analogen CW-Dopplersignale konfiguriert sind.

6. Ultraschallsonde (110) nach Anspruch 5,
wobei die analogen CW-Dopplersignale I-Signale und Q-Signale umfassen, und
wobei die zweite Vielzahl von Leitern Folgendes umfasst:
einen ersten Leiter (398), der zum Übertragen der I-Signale konfiguriert ist; und
einen zweiten Leiter (399), der zum Übertragen der Q-Signale konfiguriert ist.

7. Ultraschallsonde (110) nach Anspruch 6, weiter umfassend:
eine Vielzahl von analogen I/Q-Mischern (382, 384), die im Gehäuse (305) angeordnet sind, wobei die Vielzahl von analogen I/Q-Mischern jeweils einer Vielzahl von Empfangselementen des Wandlerarrays (112) entsprechen.

8. Ultraschallsonde (110) nach Anspruch 7, wobei entweder:
der erste Leiter (398) und der zweite Leiter (399) elektrisch parallel mit der Vielzahl von analogen I/Q-Mischern (382, 384) gekoppelt sind;
oder
jeweilige Ausgänge der Vielzahl von analogen I/Q-Mischern (382, 384) so summiert werden, dass der erste Leiter (398) und der zweite Leiter (399) summierte Ausgänge von analogen I/Q-Mischern übertragen.

9. Ultraschallsonde (110) nach Anspruch 1, weiter umfassend:
einen Quadratur-Taktgenerator (380), der in dem Gehäuse (305) angeordnet ist und mit den analogen I/Q-Mischern (382, 384) in Kommunikation steht.

10. Ultraschallsonde (110) nach Anspruch 9, wobei das Kabel (390) eine Vielzahl von Leitern (394, 391, 392) umfasst, die dazu konfiguriert sind, Strom-, Takt- und Steuersignale vom Ultraschallsystem (130) zum Quadratur-Taktgenerator (380) zu übertragen.

11. Ultraschallsonde (110) nach Anspruch 1, weiter umfassend:
einen analogen Strahlformer (314), der im Gehäuse (305) angeordnet ist und mit der Wandleranordnung (112) in Kommunikation steht.

12. Einrichtung (100), umfassend:
die Ultraschallsonde (110) nach Anspruch 1; und
das Ultraschallsystem (130), wobei das Ultraschallsystem so von der Ultraschallsonde beabstandet ist, dass sich das Kabel (390) zwischen der Ultraschallsonde und dem Ultraschallsystem erstreckt.

13. Einrichtung (100) nach Anspruch 12, wobei das Ultraschallsystem (130) eine Prozessorschaltung (210) umfasst, die konfiguriert ist zum:
Erzeugen einer grafischen Darstellung einer Verteilung der Blutflussgeschwindigkeiten auf der Grundlage der analogen CW-Doppler-Signale; und
Ausgeben der grafischen Darstellung an eine Anzeige (466), die mit der Prozessorschaltung in Kommunikation steht.

14. Einrichtung (100) nach Anspruch 13,
wobei die Ultraschallsonde (110) dazu konfiguriert ist, die analogen Ultraschallsignale (160) in digitale Ultraschallsignale (168) umzuwandeln,
wobei das Kabel (390) dazu konfiguriert ist, die digitalen Ultraschallsignale von der Ultraschallsonde an das Ultraschallsystem zu übertragen, und wobei die Prozessorschaltung (210) konfiguriert ist zum:
Erzeugen eines Ultraschallbildes eines Herzens auf der Grundlage der digitalen Ultraschallsignale; und
Ausgeben des Ultraschallbildes an die Anzeige (466).

15. Verfahren (600), umfassend:
Erzeugen (605) analoger Ultraschallsignale mit einem Wandlerarray einer Ultraschallsonde;
Erzeugen (610) analoger CW-Dopplersignale auf der Grundlage der analogen Ultraschallsignale mit analogen In-Phase-&-Quadrature- (I/Q)- Mischern, die in einem Gehäuse der Ultraschallsonde angeordnet sind;
Übertragen (615) der analogen CW-Dopplersignale von der Ultraschallsonde zu einem Ultraschallsystem, das durch ein mit dem Gehäuse gekoppeltes Kabel von der Ultraschallsonde beabstandet ist;
Erzeugen (620) einer grafischen Darstellung der Blutflussgeschwindigkeiten auf der Grundlage der analogen CW-Dopplersignale mit einer Prozessorschaltung des Ultraschallsystems; und
Ausgeben (625) der grafischen Darstellung an eine Anzeige in Kommunikation mit der Prozessorschaltung.

## Revendications

1. Sonde à ultrasons (110) conçue pour communiquer avec un système à ultrasons (130), la sonde à ultrasons comprenant :
un réseau de transducteurs (112) configuré pour générer des signaux ultrasonores analogiques (160) ;
des mélangeurs (382, 384) analogiques en phase/en quadrature (I/Q) disposés à l'intérieur d'un boîtier (305) de la sonde à ultrasons et en communication avec le réseau de transducteurs, dans laquelle les mélangeurs analogiques I/Q sont configurés pour générer des signaux Doppler analogiques à onde entretenue (CW) basés sur les signaux ultrasonores analogiques ; et
un câble (390) couplé au boîtier, dans laquelle le câble est configuré pour transmettre les signaux Doppler CW analogiques de la sonde à ultrasons au système à ultrasons.

2. Sonde à ultrasons (110) selon la revendication 1, comprenant en outre :
un convertisseur analogique-numérique (ADC) (320) disposé à l'intérieur du boîtier (305) et en communication avec le réseau de transducteurs (112), dans laquelle l'ADC est configuré pour convertir les signaux ultrasonores analogiques (160) en signaux ultrasonores numériques (168) et dans laquelle le câble (390) est configuré pour transmettre les signaux ultrasonores numériques au système à ultrasons (130).

3. Sonde à ultrasons (110) selon la revendication 2, comprenant en outre :
au moins un formateur de faisceaux numériques ou un multiplexeur en communication avec l'ADC (320).

4. Sonde à ultrasons (110) selon la revendication 2, dans laquelle le câble (390) comprend une première pluralité de conducteurs (396) configurés pour transmettre les signaux ultrasonores numériques.

5. Sonde à ultrasons (110) selon la revendication 4, dans laquelle le câble (390) comprend une seconde pluralité de conducteurs (398, 399) configurés pour transmettre les signaux Doppler CW analogiques.

6. Sonde à ultrasons (110) selon la revendication 5,
dans laquelle les signaux Doppler CW analogiques comprennent des signaux I et des signaux Q, et
dans laquelle la seconde pluralité de conducteurs comprend :
un premier conducteur (398) configuré pour transmettre les signaux I ; et
un second conducteur (399) configuré pour transmettre les signaux Q.

7. Sonde à ultrasons (110) selon la revendication 6, comprenant en outre :
une pluralité de mélangeurs analogiques I/Q (382, 384) disposés à l'intérieur du boîtier (305), dans laquelle la pluralité de mélangeurs analogiques I/Q correspondent respectivement à une pluralité d'éléments de réception du réseau de transducteurs (112).

8. Sonde à ultrasons(110) selon la revendication 7, dans laquelle soit :
le premier conducteur (398) et le second conducteur (399) sont couplés électriquement à la pluralité de mélangeurs analogiques I/Q (382, 384) en parallèle ;
soit
des sorties respectives de la pluralité de mélangeurs analogiques I/Q (382, 384) sont additionnées de telle sorte que le premier conducteur (398) et le second conducteur (399) émettent des sorties additionnées de mélangeurs analogiques I/Q.

9. Sonde à ultrasons (110) selon la revendication 1, comprenant en outre :
un générateur d'horloge en quadrature (380) disposé à l'intérieur du boîtier (305) et en communication avec les mélangeurs analogiques I/Q (382, 384).

10. Sonde à ultrasons (110) selon la revendication 9, dans laquelle le câble (390) comprend une pluralité de conducteurs (394, 391, 392) configurés pour transmettre des signaux d'alimentation, d'horloge et de commande du système à ultrasons (130) au générateur d'horloge en quadrature (380).

11. Sonde à ultrasons (110) selon la revendication 1, comprenant en outre :
un formateur de faisceaux analogiques (314) disposé à l'intérieur du boîtier (305) et en communication avec le réseau de transducteurs (112).

12. Appareil (100) comprenant :
la sonde à ultrasons (110) selon la revendication 1; et
le système à ultrasons (130), dans lequel le système à ultrasons est espacé de la sonde à ultrasons de telle sorte que le câble (390) s'étende entre la sonde à ultrasons et le système à ultrasons.

13. Appareil (100) selon la revendication 12, dans lequel le système à ultrasons (130) comprend un circuit de processeur (210) configuré pour :
générer une représentation graphique d'une distribution des vitesses de flux sanguin sur la base des signaux Doppler CW analogiques ; et
produire en sortie la représentation graphique sur un écran (466) en communication avec le circuit de processeur.

14. Appareil (100) selon la revendication 13,
dans lequel la sonde à ultrasons (110) est configurée pour convertir les signaux ultrasonores analogiques (160) en signaux ultrasonores numériques (168),
dans lequel le câble (390) est configuré pour transmettre les signaux ultrasonores numériques de la sonde à ultrasons au système à ultrasons, et dans lequel le circuit de processeur (210) est configuré pour :
générer une image ultrasonore d'un cœur sur la base des signaux ultrasonores numériques ; et
produire en sortie l'image ultrasonore sur l'écran (466).

15. Procédé (600) comprenant :
la génération (605) de signaux ultrasonores analogiques avec un réseau de transducteurs d'une sonde à ultrasons ;
la génération (610) de signaux Doppler CW analogiques sur la base des signaux ultrasonores analogiques avec des mélangeurs analogiques en phase/en quadrature (I/Q) disposés à l'intérieur d'un boîtier de la sonde à ultrasons ;
la transmission (615) des signaux Doppler CW analogiques de la sonde à ultrasons à un système à ultrasons espacé de la sonde à ultrasons par un câble couplé au boîtier ;
la génération (620), avec un circuit de processeur du système à ultrasons, d'une représentation graphique des vitesses de flux sanguin sur la base des signaux Doppler CW analogiques ; et
la production en sortie (625) de la représentation graphique vers un dispositif d'affichage en communication avec le circuit de processeur.
